# EUROPÄISCHE PATENTANMELDUNG

(11) **EP 1 043 575 A2**
(43) Veröffentlichungstag der Anmeldung: **11.10.2000**
(21) Anmeldenummer: 00101046.1
(22) Anmeldetag: 20.01.2000
(51) Int. Cl.: G01J 3/04, G02B 5/00

(54) **Baugruppe zur Erzeugung eines optisch wirksamen Spaltes**

(30) Priorität: 12.03.1999 DE 19910942
(71) Anmelder: CARL ZEISS JENA GMBH, 07745 JENA (DE)
(72) Erfinder: Koschmieder, Ingo, Dipl.-Phys., 07743 Jena (DE); Link, Günter, Dipl.-Ing., 07747 Jena (DE); Steinmetz, Dietmar, Dipl.-Ing., 07751 Bucha (DE); Luther, Egon, Dipl.-Ing., 07751 Cospeda (DE)
(74) Vertreter: Hampe, Holger

(57) **Zusammenfassung**

Die Erfindung bezieht sich auf eine Baugruppe zur Erzeugung eines optisch wirksamen Spaltes (1) mit veränderbarer Spaltbreite s, bevorzugt für Spaltlampen, mit zwei an einer Geradführung gegeneinander verschiebbaren Spaltbacken (2.1,2.2), zwischen denen der Spalt (1) ausgebildet ist, wobei jeder Spaltbacke (2.1,2.2) eine von zwei Führungsbahnen (10.1,10.2) zugeordnet ist.

Bei einer Baugruppe zur Erzeugung eines optisch wirksamen Spaltes der vorgenannten Art ist vorgesehen, daß die Führungsbahnen (10.1,10.2) an Abschnitten einer Führungsschiene (7) ausgebildet sind, die von einer gestellfesten Einspannposition ausgehend in die Verschieberichtungen der Spaltbacken (2.1,2.2) frei vorstehen. Die Führungsschiene (7) kann sich so unter Temperatureinfluß in Verschieberichtung ungehindert ausdehnen, ohne daß dadurch die Spaltparallelität beeinflußt wird.

## Beschreibung

Die Erfindung bezieht sich auf eine Baugruppe zur Erzeugung eines optisch wirksamen Spaltes mit veränderbarer Spaltbreite s, bevorzugt für Spaltlampen, mit zwei an einer Geradführung gegeneinander verschiebbaren Spaltbacken, zwischen denen der Spalt ausgebildet ist, wobei jeder Spaltbacke eine von zwei Führungsbahnen zugeordnet ist.

Im optischen Gerätebau ist es häufig erforderlich, Leuchtfelder unterschiedlicher geometrischer Gestalt bei einer nahezu gleichmäßigen Ausleuchtung zu erzeugen. Eine sehr häufig benötigte Leuchtfeldform ist ein in Breite und Länge verstellbarer Lichtspalt mit exakt paralleler Begrenzung der Spaltbreite. Die Erzeugung eines solchen Lichtspaltes mit kontrastreichen scharfen Bildgrenzen setzt eine Präzisionsführung der spaltformenden Bauteile und eine hohe Kantenqualität an den Spaltbacken voraus.

Ein bevorzugter Anwendungsfall für Baugruppen zur Erzeugung eines optisch wirksamen Spaltes ist die Spaltlampe, die häufig zur ophthalmologischen Diagnose eingesetzt wird. Sie besteht im wesentlichen aus einem Stereomikroskop und einer Projektionseinrichtung, die beide in Koordination zueinander bewegbar auf einem Tragesystem angeordnet sind. Die Projektionseinrichtung ermöglicht es, geometrisch unterschiedliche Lichtfelder zu erzeugen. Sie ist mit dem Stereomikroskop so gekoppelt, daß die Mitte der Leuchtfeldebene in der Regel mit der Mitte der Beobachtungsebene zusammenfällt.

Für derartige Anwendungen wird beispielsweise eine von "Null" bis 14 mm stufenlos und feinfühlig einstellbare Spaltbreite gefordert, wobei "Null" ein absolut dichtes Schließen des Spaltes bedeutet. Die Länge des Spaltes kann beispielsweise zwischen 0,3 mm und 14 mm betragen, und häufig muß der Spalt außerdem um mindestens ±90° um die optische Achse verschwenkbar sein.

Ein wesentliches Problem bei spaltbildenden optischen Baugruppen besteht immer wieder darin, daß die funktionell bedingte Nähe des Lampenhauses, welches die wärmeabgebende Beleuchtungsquelle enthält, Temperaturschwankungen bewirkt, die sich nachteilig auf die Spaltparallelität auswirken und eine Spaltbackenführung erfordern, die trotz des Temperatureinflusses einen Lichtspalt erzeugen, der den Qualitätsansprüchen genügt.

Die nach dem Stand der Technik bekannten Spaltbackenführungen weisen Geradführungselemente auf der Basis von Gleit- oder Rollreibung auf, bei denen zwei zur Veränderung der Spaltbreite gegeneinander verschiebbare Spaltbacken über entsprechend ausgebildete Gleit- oder Rollführungselemente gegen eine gestellseitig angeordnete Führungsbahn spielfrei vorgespannt sind.

Nachteil bei allen bekannten technischen Lösungen in diesem Zusammenhang ist die immer noch in unerwünscht hohem Maße zu verzeichnende Übertragung von thermischen und mechanischen Einflüssen auf die Spaltbacken und die damit verbundene unerwünschte Beeinflussung der Spaltparallelität, was sich beispielsweise dahingehend bemerkbar macht, daß der Spalt nicht absolut dicht geschlossen werden kann. Insofern gibt es trotz der bereits bekannten Vielzahl von Lösungen immer wieder Entwicklungsbedarf, wobei das Entwicklungsziel auf die Reduzierung der störenden Einflüsse bzw. deren Auswirkungen gerichtet ist.

Ausgehend von diesem Stand der Technik liegt der Erfindung die Aufgabe zugrunde eine Baugruppe der vorgenannten Art zur Erzeugung eines optisch wirksamen Spaltes so weiterzubilden, daß die Auswirkungen von äußeren Einflüsse auf die Parallelität des Lichtspaltes weiter verringert werden.

Erfindungsgemäß ist bei einer Baugruppe zur Erzeugung eines optisch wirksamen Spaltes der vorgenannten Art vorgesehen, daß die Führungsbahnen an Abschnitten einer Führungsschiene ausgebildet sind, die von einer gestellfesten Einspannposition ausgehend in die Verschieberichtungen der Spaltbacken frei vorstehen.

Durch das freie Vorstehen der Führungsschiene wird erreicht, daß sich diese unter Temperatureinfluß in Verschieberichtung ungehindert ausdehnen kann, ohne daß dadurch die Spaltparallelität beeinflußt wird.

In einer bevorzugten Ausgestaltungsvariante der Erfindung ist vorgesehen, daß die Führungsbahnen an zwei von einer Einspannposition in entgegengesetzte Richtungen freitragend vorstehenden Abschnitten einer Führungsschiene ausgebildet sind. Damit ist der Ausgleich von Längenänderung durch ungehinderte Längenausdehnung dieser beiden Abschnitte nach entgegengesetzten Richtungen hin möglich.

In einer anderen Ausgestaltungsvariante der Erfindung ist vorgesehen, daß die Führungsbahnen an zwei Abschnitten der Führungsschiene ausgebildet sind, die von einer Einspannposition ausgehend in eine Richtung vorstehen, wobei der der Einspannposition gegenüberliegende Endabschnitt der Führungsschiene schwimmend gelagert ist. Hierdurch ist der Ausgleich von Längenänderungen der Führungsschiene von der Einspannposition aus nach dieser Richtung hin gewährleistet. Die Führungsschiene kann sich in Verschieberichtung ausdehnen, während ihre Positionsveränderung senkrecht zur Verschieberichtung nicht möglich ist.

Weiterhin kann vorteilhaft vorgesehen sein, daß in der Mitte zwischen der Einspannposition und dem gegenüberliegenden, gleitend gelagerten Endabschnitt Mittel zur Durchbiegung der Führungsschiene vorhanden sind, wobei mit der Durchbiegung die Fluchtung der beiden Führungsbahnen korrigierbar ist. Wird die Schiene mit Hilfe dieser Mittel durchgebogen und dabei die Fluchtung der beiden Führungsbahnen zueinander justiert, kann auf diese Weise die Parallelität des Spaltes beeinflußt werden. Somit ist eine Justiermöglichkeit geben, die bevorzugt zu einer optimalen Einstellung der Spaltparallelität vor der Montage der erfindungsgemäßen Baugruppe in eine optischen Einrichtung, beispielsweise in eine Spaltlampe, vorgenommen werden kann.

In diesem Zusammenhang besteht eine besonders bevorzugte Ausgestaltung darin, daß als Mittel zur Durchbiegung der Führungsschiene ein Gewindestift vorgesehen ist, bei dessen Zustellung eine Durchbiegung der Führungsschiene in Längsrichtung des Spaltes erzielt wird. Dabei sollten die Führungsschiene, die Einspannung für die Führungsschiene und der Gewindestift aus gleichem Material mit möglichst geringem Ausdehnungskoeffizienten, bevorzugt einem Sonderstahl, gefertigt sein, so daß Längenänderungen, die ihre Ursache in Temperatureinflüssen haben, kompensiert werden.

In einer weiteren Ausgestaltung der Erfindung ist vorgesehen, daß den Führungsbahnen Schieber gegenüberstehen, die entweder mit Gleit- oder Wälzkörperführungen ausgestattet sind und an denen die Spaltbacken befestigt sind. Die Wälzkörperführungen können beispielsweise derart ausgestaltet sein, daß sie die Führungsbahnen umgreifen.

Alternativ hierzu kann aber auch vorgesehen sein, daß an die Schieber V-Nuten angearbeitet sind, die gegen die Führungsbahnen gleiten. In diesem Zusammenhang ist es vorteilhaft, wenn Zug- und/oder Druckfedern zur Erzeugung einer Vorspannkraft zwischen den Schiebern und den Führungsbahnen vorhanden sind, wobei jeweils ein Federende gestelltest und das andere Federende an einem der Schieber eingehängt ist. Damit ist gewährleistet, daß die V-Nuten stets Kontakt zu den zugeordneten Führungsbahnen haben und so stets auch in Führungsrichtung ausgerichtet sind.

In einer Ausgestaltung sind hier Zugfedern vorgesehen und diese so angeordnet, daß deren Federkraft mit einer ersten Komponente F₁ die Vorspannung der V-Nuten gegen die jeweils zugeordnete Führungsbahn und mit einer zweiten Komponente F₂ eine Vorspannung des jeweiligen Schiebers gegen ein Stellglied bewirkt, das zur Einstellung eines Abstandes zwischen den Schiebern in Verschieberichtung und damit zur Einstellung dient. Auf diese Weise wird erreicht, daß mit jeweils einer einem Schieber bzw. einer Spaltbacke zugeordneten Feder sowohl die Anlage der V-Nuten gegen die Führungsbahn als auch die Anlage des Schiebers gegen das Stellglied gewährleistet ist. Zusätzlich können die Zugfedern so angeordnet sein, daß die Federkraft mit einer dritten Komponente F₃ eine Drehung der Spaltbacken um die Verschieberichtung verhindert, wie im Ausführungsbeispiel noch ausführlich dargestellt wird.

Vorteilhaft können zur Minimierung der Reibung zwischen dem Stellglied und den Schiebern Wälzlager angeordnet sein, über die die Verstellbewegung vom Stellglied auf die Schieber übertragen wird. Mit der Reduzierung der Reibung an dieser Stelle wird eine Verringerung der Kippmomente erreicht, die bei Drehung des Stellgliedes auf die Schieber gerichtet sind und damit die Gefahr einer Verstellung der Spaltparallelität in sich bergen.

Besonders vorteilhaft ist es, wenn die Einhängung der Zugfedern an den Schiebern zumindest etwa in der Achse der Wälzlagerung vorgesehen ist, die zur Verminderung der Reibung zwischen den Schiebern und dem Stellglied dienen.

Um eine optimale Gleitpaarung zwischen den V-Nuten und den Führungsbahnen zu erzielen, können die Oberflächen der V-Nuten und/oder der Führungsbahnen mit einer reibungsmindernden Beschichtung, beispielsweise mit einer DLC-Schicht, versehen sein.

Die Erfindung soll nachfolgend anhand von zwei Ausführungsbeispielen näher erläutert werden. In den dazugehörigen Zeichnungen zeigen
- Fig.1: die Prinzipdarstellung einer ersten Ausgestaltungsvariante in einer Draufsicht
- Fig.2: einen Schnitt AA aus Fig.1
- Fig.3: einen Schnitt BB aus Fig.2
- Fig.4: die Prinzipdarstellung einer zweiten Ausgestaltungsvariante
- Fig.5: einen Schnitt CC aus Fig.4

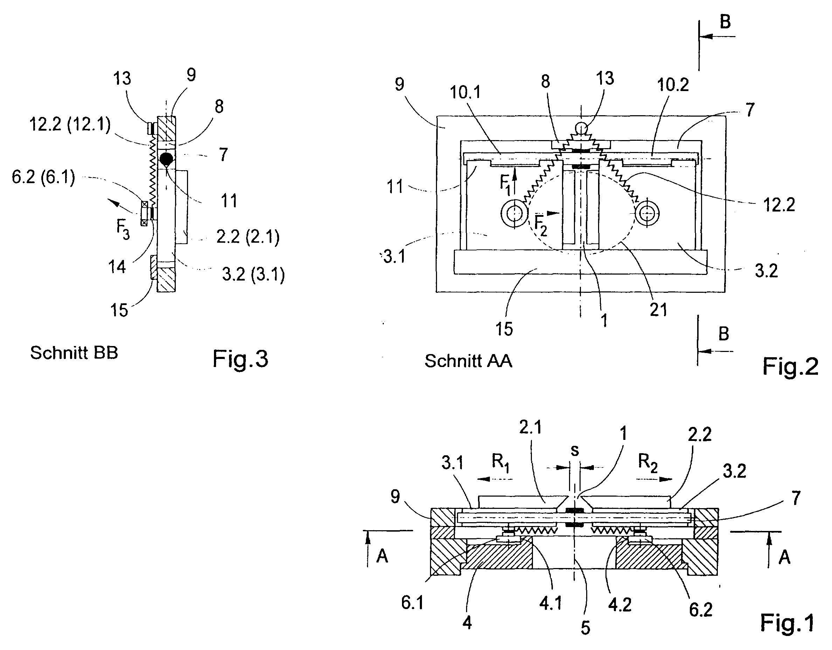

In Fig.1 ist die erfindungsgemäße Baugruppe zur Erzeugung eines optisch wirksamen Spaltes 1 mit veränderbarer Spaltbreite s dargestellt. Der Spalt 1 ist zwischen zwei Spaltbacken 2.1 und 2.2 ausgebildet, die in den Richtungen R₁ und R₂ verschiebbar angeordnet sind.

Die Spaltbacken 2.1 und 2.2 stehen über zugeordnete Schieber 3.1 und 3.2, an denen sie befestigt sind, mit einem Stellglied 4 in Verbindung, das mit Ellipsenstellflächen 4.1 und 4.2 versehen und um eine Drehachse 5 verdrehbar gelagert ist. Mit der Verdrehung des Stellgliedes 4 um die Drehachse 5 können die Abstände zwischen den Ellipsenstellflächen 4.1 und 4.2 und der Drehachse 5 geändert werden.

Wird das Stellglied 4 (z.B. manuell) so gedreht, daß sich diese Abstände vergrößern, wird diese Abstandsänderung über die Außenringe, die Wälzkörper und die (mit den Schiebern 3.1 bzw. 3.2 fest verbunden) Innenringe der symbolisch dargestellten Kugellager 6.1 und 6.2 auf die Schieber 3.1 und 3.2 übertragen, wodurch letztere ihren Abstand zueinander verändern und die Breite s des Spaltes 1 sich vergrößert.

Um die Parallelität der Spaltbacken 2.1,2.2 zu gewährleisten, ist eine präzise Geradführung der Schieber 3.1,3.2 erforderlich. Diesem Zweck dient eine Führungsschiene 7, die mit Hilfe einer Spanneinrichtung 8 (vgl. Fig.2) an einem Rahmen 9 gestellfest positioniert ist. Wie in Fig.2 erkennbar, sind an der Führungsschiene 7 zwei gleichlange Führungsbahnen 10.1 und 10.2 ausgebildet, die in entgegengesetzten Richtungen freitragend von der Einspannposition auskragen.

Die Schieber 3.1,3.2 können alternativ sowohl über Gleit- als auch über Wälzpaarungen mit den Führungsbahnen 10.1,10.2 verbunden sein; entsprechend einer Ausgestaltungsvariante wird die Erfindung im folgenden anhand einer Gleitpaarung erläutert.

Hierbei weisen die Führungsbahnen 10.1,10.2 kreisrunde Querschnitte auf, während an den Schiebern 3.1,3.2 V-Nuten vorgesehen sind, die gegen die Führungsbahnen 10.1,10.2 gleiten. Eine verhältnismäßig reibungsarme Gleitpaarung erhält man beispielsweise, wenn die Oberfläche der V-Nuten und/oder der Führungsbahnen 10.1,10.2 mit einer DLC-Schicht versehen sind. Der Nutgrund 11 der V-Nuten ist in Fig.2 als gestrichelte Linie dargestellt.

In Fig.2 sind als Seitenansicht weiterhin die Schieber 3.1,3.2, die Führungsstange 7 mit den Führungsbahnen 10.1,10.2 und die Spanneinrichtung 8 zu erkennen, in welche die Führungsstange 7 mittig eingespannt ist.

In Fig.3 (einem Schnitt BB aus Fig.2) ist der kreisrunde Querschnitt der Führungsschiene 7 zu erkennen. Diese ist in die Spanneinrichtung 8 (mit nahezu spielfreier Gleitpassung) eingelassen und kann hier mit einer Spannschraube (nicht dargestellt) geklemmt werden.

Um die Anlage der V-Nuten an die Führungsbahnen 10.1,10.2 zu gewährleisten, sind Zugfedern 12.1 und 12.2 vorgesehen, von denen jede mit einem ihrer Enden an einem Bolzen 13 (siehe Fig.2 und Fig.3) eingehängt ist. Der Bolzen 13 ist am Rahmen 9 und damit gestellfest angeordnet. Mit ihren gegenüberliegenden Enden sind die Zugfedern 12.1,12.2 an Bolzen 14 eingehängt, die etwa im geometrischen Zentrum der Schieber 3.1,3.2 positioniert sind. Auf den Bolzen 14 sind auch die Innenringe der Kugellager 6.1,6.2 angeordnet.

Damit wirkt jede der beiden Zugfedern 12.1,12.2 mit einer Kraftkomponente F₁ in Richtung zur Führungsschiene 7 und veranlaßt so für jeden der beiden Schieber 3.1,3.2 die sichere Anlage der V-Nut an die zugeordnete Führungsbahn 10.1,10.2. Außerdem wirkt jeweils eine Federkraftkomponente F₂ in Verschieberichtung der Schieber 3.1,3.2, und zwar so, daß die Wirkungsrichtung der Komponenten F₂ den Verstellrichtungen R₁ und R₂ (vgl. Fig.1) entgegengesetzt und somit die Anlage der Außenringe der Kugellager 6.1,6.2 gegen die Ellipsenstellflächen 4.1,4.2 gesichert ist. Die Ellipsenstellflächen 4.1,4.2 am Stellglied 4 bilden in der Seitenansicht eine elliptische Kurve 15, die in Fig.2 als gestrichelte Linie dargestellt ist.

Wird das Stellglied 4 so gedreht, daß sich die Abstände der Ellipsenstellflächen 4.1,4.2 zur Drehachse 5 verkleinern, so wirken die Komponenten F₂ als Rückstellkräfte, durch die die Schieber 3.1,3.2 entgegengesetzt zu den Richtungen R₁ und R₂ verschoben werden und die Breite s des Spalten 1 verringert wird.

Die Federkonstante der beiden Zugfedern 12.1,12.2 sollte so gewählt sein, daß die Kraftkomponenten F₁ in allen Verschiebepositionen der Schieber 3.1,3.2 etwa gleich groß sind. Ein wesentlicher Vorteil einer solchen Anordnung besteht darin, daß die Federkomponenten F₁ und F₂ symmetrisch zu den Spaltbacken 2.1,2.2 angreifen und somit deren Verkanten ausgeschlossen ist.

In Fig.3 ist schließlich auch zu erkennen, daß die Kraftwirkungen der Zugfedern 12.1,12.2 so gerichtet sind, daß die Schieber 3.1,3.2 mit einem durch eine Kraftkomponente F₃ hervorgerufenen Kippmoment um die Führungsbahnen 10.1,10.2 beaufschlagt sind, das durch eine Anschlagleiste 15 aufgefangen wird. Damit wird eine Verdrehung der Schieber 3.1,3.2 um die Verschieberichtung verhindert.

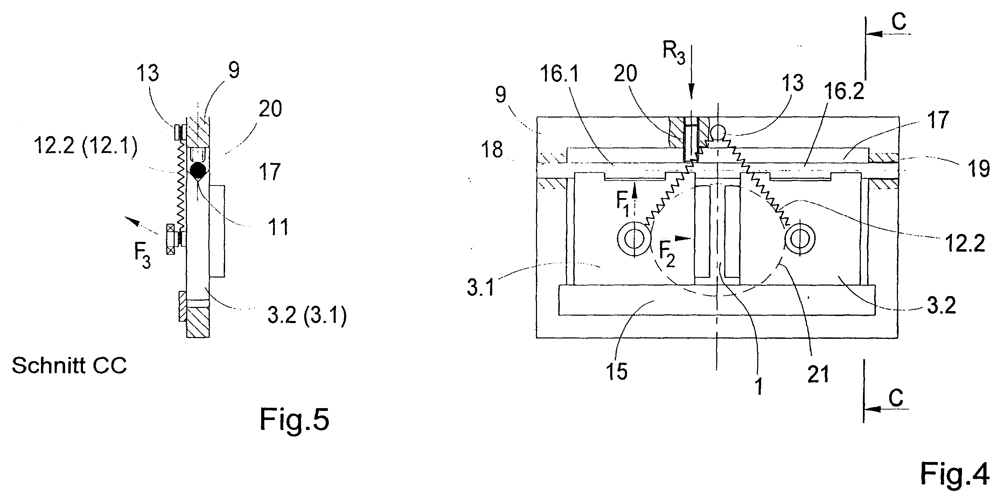

In einer von dem bis hierher dargestellten Beispiel abweichenden Ausgestaltung der Erfindung nach Fig.4 sind Führungsbahnen 16.1 und 16.2 an zwei Abschnitten einer Führungsschiene 17 vorgesehen, die an einer Einspannposition 18 gestellfest (z.B. durch Preßpassung) gehalten ist und die von dieser Einspannposition 18 in eine Richtung in den Rahmen 9 hinein auskragt.

Diesbezüglich ist aus Fig.4 ersichtlich, daß der der gestellfesten Einspannung gegenüberliegende Endabschnitt der Führungsschiene 17 in einer Gleitpassung 19 verschieblich gelagert ist. Mit dieser Anordnung ist eine durch Temperatureinflüsse bedingte Längenänderung der Führungsschiene 17 gewährleistet, da in diesem Falle eine Relativverschiebung zwischen der Führungsschiene 17 und dem Rahmen 9 innerhalb der Gleitpassung 19 erfolgt.

Den Führungsbahnen 16.1,16.2 sind wie im ersten Beispiel Schieber 3.1,3.2 zugeordnet. An diesen Führungsbahnen 16.1,16.2 wird die Geradführung der Schieber 3.1,3.2 bzw. der Spaltbacken 2.1,2.2 analog zum ersten Beispiel erzielt.

Nach Fig.4 ist der Spalt 1 rechtwinklig zur Führungsschiene 17 ausgerichtet. Insofern ist leicht vorstellbar, daß die Parallelität des Spaltes 1 (selbstverständlich eine hohe Fertigkeitsgenauigkeit aller Teile der Baugruppe vorausgesetzt) genau dann gewährleistet ist, wenn die Führungsbahnen 16.1,16.2 zueinander fluchten. Um diese Fluchtung schon vor Einbau der Baugruppe in ein optisches Gerät optimal einstellen zu können, ist in einer besonderen Ausgestaltung der Erfindung etwa in der Mitte zwischen der Einspannposition 18 und der Gleitpassung 19 ein Gewindestift 20 als Stellelement in den Rahmen 9 eingelassen, dessen Zustellrichtung durch R₃ gekennzeichnet ist. Mit der Zustellung des Gewindestiftes 20 können Abweichungen korrigiert werden, so daß mit einjustierter Fluchtung beider Führungsbahnen 16.1,16.2 auch die Parallelität des Spaltes 1 gegeben ist. Außerdem wird hierbei eine selbsttätige Kompensation von Temperatureinflüssen erreicht, wenn die Materialien für die Führungsschiene 7, den Rahmen 9 und den Gewindestift 20 im Hinblick auf gleichen und auch minimierten Ausdehnungskoeffizienten gewählt sind.

Weiterhin kann vorteilhaft vorgesehen sein, daß die V-Nuten nicht durchgängig an den Schiebern 3.1,3.2 ausgebildet, sondern zwischen ihren Enden freigefräst sind, wodurch einem Kippen der Schieber 3.1,3.2 in Verschieberichtung vorgebeugt wird.

Fig.5 zeigt den Angriff des Gewindestiftes 20 auf die Führungsschiene 17 in einer Seitenansicht aus Fig.4.

### Bezugszeichenliste

- 1: Spalt
- 2.1, 2.2: Spaltbacken
- 3.1, 3.2: Schieber
- 4: Stellglied
- 4.1, 4.2: Ellipsenstellflächen
- 5: Drehachse
- 6.1, 6.2: Kugellager
- 7: Führungsschiene
- 8: Spanneinrichtung
- 9: Rahmen
- 10.1,10.2: Führungsbahnen
- 11: Nutgrund
- 12.1,12.2: Zugfedern
- 13, 14: Bolzen
- 15: Anschlagleiste
- 16.1,16.2: Führungsbahnen
- 17: Führungsschiene
- 18: Einspannposition
- 19: Gleitpassung
- 20: Gewindestift
- 21: Kurve

## Patentansprüche

1. Baugruppe zur Erzeugung eines optisch wirksamen Spaltes mit veränderbarer Spaltbreite s, mit zwei an einer Geradführung gegeneinander verschiebbaren Spaltbacken, zwischen denen der Spalt ausgebildet ist, wobei jeder Spaltbacke eine von zwei Führungsbahnen zugeordnet ist, **dadurch gekennzeichnet**, daß die Führungsbahnen an Abschnitten einer Führungsschiene ausgebildet sind, die von einer gestellfesten Einspannposition ausgehend in die Verschieberichtungen der Spaltbacken frei vorstehen.

2. Baugruppe nach Anspruch 1, dadurch gekennzeichnet, daß die Führungsbahnen (10.1,10.2) an zwei Abschnitten einer Führungsschiene (7) ausgebildet sind, die von einer Spanneinrichtung (8) ausgehend in einander entgegengesetzte Richtungen freitragend vorstehen.

3. Baugruppe nach Anspruchs 1, dadurch gekennzeichnet, daß die Führungsbahnen (16.1,16.2) an zwei Abschnitten einer Führungsschiene (17) ausgebildet sind, die von einer Einspannposition (18) ausgehend in eine Richtung vorstehen, wobei der der Einspannposition (18) gegenüberliegende Endabschnitt der Führungsschiene (17) schwimmend gelagert ist.

4. Baugruppe nach Anspruch 3, dadurch gekennzeichnet, daß mittig zwischen der Einspannposition (18) und dem gegenüberliegenden schwimmend gelagerten Endabschnitt der Führungsschiene (17) Mittel zur Durchbiegung der Führungsschiene (17) vorhanden sind, wobei mit der Durchbiegung die Fluchtung der beiden Führungsbahnen (16.1,16.2) beeinflußbar ist.

5. Vorrichtung nach Anspruch 4, dadurch gekennzeichnet, daß als Mittel zur Durchbiegung ein Gewindestift (20) vorgesehen ist, wobei Gewindestift (20) und Führungsschiene (7) aus Material mit gleichem Ausdehnungskoeffizienten gefertigt sind.

6. Baugruppe nach einem der vorgenannten Ansprüche, dadurch gekennzeichnet, daß den Führungsbahnen (10.1,10.2;16.1,16.2) Schieber (3.1,3.2) gegenüberstehen, die mit Gleit- oder Wälzkörperführungen ausgestattet sind und an denen die Spaltbacken (2.1,2.2) befestigt sind.

7. Baugruppe nach Anspruch 6, dadurch gekennzeichnet, daß an den Schiebern (3.1,3.2) Wälzkörperführungen vorgesehen sind, die die Führungsbahnen (10.1,10.2;16.1,16.2) umgreifen.

8. Baugruppe nach Anspruch 6, dadurch gekennzeichnet, daß an den Schiebern (3.1,3.2) V-Nuten vorgesehen sind, die gegen die Führungsbahnen (10.1,10.2;16.1,16.2) gleiten.

9. Baugruppe nach Anspruch 8, dadurch gekennzeichnet, daß Zug- und/oder Druckfedern zur Erzeugung einer Vorspannkraft zwischen den Schiebern (3.1,3.2) und den Führungsbahnen (10.1,10.2;16.1,16.2) vorhanden sind, wobei jeweils ein Federende gestellfest und das andere Federende an einem der Schieber (3.1,3.2) eingehängt ist.

10. Baugruppe nach Anspruch 9, dadurch gekennzeichnet, daß Zugfedern (12.1,12.2) vorgesehen und so angeordnet sind, daß die Federkraft mit einer ersten Komponente F₁ die Vorspannung der Schieber (3.1,3.2) gegen die Führungsbahnen (10.1,10.2;16.1,16.2) und mit einer zweiten Komponente F₂ eine Vorspannung der Schieber (3.1,3.2) gegen an einem Stellglied (4) ausgebildete Ellipsenstellflächen (4.1,4.2) bewirkt, die zur Einstellung eines Abstandes zwischen den Schiebern (3.1,3.2) in Verschieberichtung und damit zur Einstellung der Spaltbreite s vorgesehen sind.

11. Baugruppe nach Anspruch 10, dadurch gekennzeichnet, daß die Zugfedern (12.1,12.2) so angeordnet sind, daß die Federkraft mit einer dritten Komponente F₃ die Vorspannung der Schieber (3.1,3.2) gegen eine Anschlagleiste (15) bewirkt, wodurch eine Drehung der Spaltbacken (2.1,2.2) um die Verschieberichtung verhindert ist.

12. Baugruppe nach Anspruch 10 oder 11, dadurch gekennzeichnet, daß zur Aufnahme der Vorspannkraft bzw. zur Minimierung der Reibung zwischen den Ellipsenstellflächen (4.1,4.2) und den Schiebern (3.1,3.2) Wälzlager (6.1,6.2) vorgesehen sind.
